# EUROPEAN PATENT SPECIFICATION

(11) **EP 3 203 834 B1**
(45) Date of publication and mention of the grant of the patent: **26.02.2020**
(21) Application number: 15781164.7
(22) Date of filing: 25.09.2015
(51) Int. Cl.: A01L 15/00, A61D 7/00, A61D 11/00, A01K 1/12, A01J 5/007

(54) **HOOF TREATMENT ARRANGEMENT**
HUFBEHANDLUNGSANORDNUNG
AGENCEMENT DE TRAITEMENT DE SABOT

(30) Priority: 06.10.2014 SE 1451183
(43) Date of publication of application: 16.08.2017
(73) Proprietor: DeLaval Holding AB, 147 21 Tumba (SE)
(72) Inventor: HALLÉN SANDGREN, Charlotte, 147 21 Tumba (SE)
(74) Representative: Lilliehorn, Tobias
(86) International application number: PCT/SE2015/051002
(87) International publication number: WO 2016/056975

(56) References cited:
- EP-A1- 1 099 373
- EP-A2- 1 384 400
- WO-A1-02/065833
- NL-C2- 1 017 154
- US-A1- 2005 076 840

## Description

### TECHNICAL FIELD OF THE INVENTION

The present invention generally relates to dairy farming, and in particular the invention relates to hoof treatments.

### DESCRIPTION OF RELATED ART AND BACKGROUND OF THE INVENTION

It is common in dairy farming to use a so called foot bath, which is usually placed where the cows exit the dairy parlor so that each cow is forced to walk through the foot bath. The foot bath normally comprises disinfectant that is dissolved in the water. An alternative to the foot bath is to use a treatment stall such as the one described in EP 1384400.

One important health issue among dairy animals is infectious hoof illnesses, particularly digital dermatitis and foot rot, which are highly contagious infections. These illnesses cause not only pain and welfare issues, but also risk for lameness and spread of bacteria within the herd of dairy animals. Early detection and treatment of these illnesses is important.

Digital dermatitis is caused by the anaerobic bacterium dichelobacter nodosus and other treponemas, and can be treated by topical antibiotics applied to the area. Liquids comprising formalin, copper sulphate and/or other chemicals may also be used for the treatment. If digital dermatitis is not treated within the first one or two weeks of the infection, it often develops into a chronic infection. This chronic infection will periodically erupt into an acute infection which does not respond to treatment but spreads the infection to the other animals in the herd. Control relies on prompt detection, isolation and treatment of affected cattle.

Foot rot is usually treated with antimicrobial products. Penicillin, tetracycline, and other antibacterial medicines are often used to treat normal cases of foot rot. It is critical to closely monitor the animals to make sure they are responding to treatment. Infected animals should be kept dry until healing has occurred. If the animal is showing no signs of recovery after three to four days, the bacteria could have infected other tissues of the foot. Infusing antibiotic into the veins of the foot may be an effective way to treat those cases. Claw amputation, and in very severe cases, culling, may also have to be considered. Foot rot may if left untreated damage the joints of the animal.

EP 1099373 describes a device for cleaning and disinfecting the feet of cows in an automatic feeding box equipped with a cow identification system. This allows for administering the correct treatment to each individual animal.

Prior art document US2005/0076840 discloses a leg treatment device provided with a device for detecting the contamination of a leg of a cow or the condition of a leg, and performing a leg treatment in dependence thereon.

### SUMMARY OF THE INVENTION

The present inventors have noted that dairy production could be improved, culling could be decreased, and animal health and welfare could be improved if the above illnesses could be detected early and treated efficiently. The inventors have come to the insight that this can be achieved by providing novel ways of determining hoof status and treating hooves in an entirely automated and efficient manner. The invention is defined in claim 1.

A first aspect of the invention refers to a hoof treatment arrangement arranged to spray a liquid onto hooves of animals while they are stationary in a stall of an animal arrangement. The hoof treatment arrangement comprises an identification system configured to identify animals, an automatic detector arrangement configured to determine the hoof status for animals, an automatic spray device, and a control arrangement. The control arrangement is operatively connected to: an information database of the animal arrangement, which comprises animal specific information for a plurality of animals housed in the animal arrangement; the identification system, to obtain information regarding the identity of each of the plurality of animals; the automatic detector arrangement, to obtain information regarding the hoof status for each of the identified animals; and the automatic spray device, to control the automatic spray device. The control arrangement is arranged to update the information database with respect to the hoof status for each of the identified animals, and configured (i) to select a subset of the plurality of animals housed in the animal arrangement based on the animal specific information and (ii) to control the automatic spray device to spray a liquid onto one or more of the hooves of only the animals of the selected subset of the plurality of animals while they are stationary in the stall of the animal arrangement.

The animal arrangement may be an enclosed area housing animals and may comprise milking, feeding, and resting facilities. The milking, feeding, and resting facilities may comprise milking, feeding, and resting stalls. The control arrangement may be a separate computer, microprocessor, circuit, or the like, but may also be an integral part of a management and/or control system responsible for management and/or control of various parts of the animal arrangement. The hoof status may e.g. be the degree of cleanliness of the hooves, or whether each of the animals has a hoof illness, such as digital dermatitis or foot rot.

Since liquid is sprayed onto one or more of the hooves of only the animals of the selected subset of the plurality of animals, the number of animal treatments are minimized and focused to those animals that require treatment as given by the animal specific information. As a consequence, animals not requiring the treatment are not treated, and the energy and liquid consumption as well as the wear and tear of the automatic spray device are minimized.

The automatic detector arrangement may comprise a camera configured to record images of one or more hooves of each of the identified animals, and image processing means configured to process the recorded images and to determine the hoof status for each of the identified animals. The camera may be carried by a robot arm configured to move the camera to appropriate positions to enable the camera to record images of one or more hooves of each of the identified animals, preferably while the animal is stationary in the stall of the animal arrangement. The image processing arrangement may comprise a computer with suitable image processing and hoof status determining software. The hoof status may e.g. include whether the animal has a hoof illness. Digital dermatitis is in its acute stage clearly visible as an approximately circular red inflammation at the back of the hoof, and therefore easy to detect using a camera. At least inflammations that are more than 20 mm in diameter should be treated. Foot rot may be more difficult to detect using just a camera, so it is preferable to use other sensors, alone or in combination with a camera. Foot rot usually causes fever and a sudden occurrence of lameness in the leg in question, and if this can be detected using a combination of sensors.

Further, the automatic spray device may also be carried by a robot arm, configured to move the automatic spray device to appropriate positions in order for the automatic spray device to spray the liquid onto one or more of the hooves of only the animals of the selected subset of the plurality of animals. In this way, cleaning can be made more forceful and thorough without wasting water, and medication can be applied more exactly where it is needed.

The robot arm may be a part of a robot already existing in the animal arrangement for other purposes such as e.g. for attachment of teat cups of a milking system. Yet alternatively, the camera of the detector arrangement and the automatic spray device are arranged on different robot arms, wherein the latter robot arm may also be provided with a camera.

The stall of the animal arrangement may e.g. be a stall of a milking system, such as an automated milking system or a rotary milking system, a stall of an animal treatment station arranged in proximity to a milking system of the animal arrangement, and into which each of the plurality of animals is passing prior, or subsequent, to the milking thereof, or a stall of an animal feeding station of the animal arrangement.

The advantages of implementing the hoof treatment arrangement in such a stall are at least twofold. Firstly it guarantees that the hoof treatment arrangement will be capable of treating all animals housed in the animal arrangement on a regular basis since all the animals visit a milking stall, a treatment stall in proximity to a milking system, and a feeding stall regularly. A high frequency of treatments can thus be obtained. This means that there is much less risk of illnesses left untreated during longer times. If all occurrences of digital dermatitis are treated before they develop into chronic infections, it is possible to greatly reduce the spread of the illness, since the chronic infections cause most of the bacteria spreading and thus the infection of the rest of the animals. Infected animals serve as a source of infection for the whole herd because they will spread the bacteria throughout the environment. The bacteria can live without a host for up to seven days. Once another animal gets a cut or crack in the soft tissue between its toes, the bacteria can infect the animal

Further, the animals tend to stand still, or at least be stationary, in such a stall, which facilitates for the hoof treatment arrangement to perform its treatment, i.e. spray a liquid onto one or more of the hooves of selected ones of the animals.

If the hoof treatment arrangement is implemented in an automated milking system having a teat cup attachment robot equipped with a camera, this camera may be the camera comprised in an automatic detector arrangement used to determine the hoof status for each of the identified animals, preferably after teat cups have been attached to that animal prior to milking thereof. In such an implementation, no additional detection hardware is required. One advantage of locating the hoof treatment arrangement in a milking stall or a treatment stall in proximity to the milking system, which the animal may e.g. enter directly after being milked, is that simultaneous separation of the animal may be performed. The animal can be separated and kept isolated, a veterinarian may be called for, or the hoof treatment may be accompanied by other kinds of treatment. At the same time, the capacity of the milking system is not affected negatively.

The separation can alternatively be performed after hoof treatment in a milking stall or a feeding stall by arranging a selection box at the exit of the stall and guiding selected animals to a separate, isolated area.

The automatic spray device may be configured to spray a cleaning liquid, such as e.g. water, onto one or more hooves of each of the identified animals prior to the detector arrangement determining the hoof status for that animal. Hereby, the hooves are cleaned and are suitably prepared for the visual hoof status detection, and the possible subsequent treatment.

According to the invention, the hoof status includes the degree of cleanliness of the hooves, and the animal specific information further includes information regarding the risk for each of the plurality of animals to become ill, which is determined based on e.g. age, race, behavior, injury status, occurrence of cuts or wounds, lactation status, illness history, or illness history of related animals. The control arrangement is then configured to select the subset of the plurality of animals housed in the animal arrangement based on the degree of cleanliness of the hooves combined with the risk for each of the plurality of animals to become ill, particularly by selecting as the subset those animals having the highest risks to become ill where the degree of cleanliness is below a threhold. The liquid is a cleaning liquid, e.g. water, or a sanitizing liquid.

The degree of cleanliness may be determined using any type of sensor which gives a different signal for a clean hoof than for a dirty hoof. If a camera is used, the image of the hoof may e.g. be compared with a reference image for the same hoof, taken when the hoof is known to be clean. Image processing may then be used to quantify the difference between the current image and the reference image, so that the degree of cleanliness can be determined. It can then be determined if the degree of cleanliness is below a predetermined threshold, which may vary in dependence on other factors, such as e.g. the infection status in the herd.

In such a manner, an entire group of animals, which may have higher risks to become ill, can be treated in a preventive manner, while other animals are not treated. It is much easier and cheaper to prevent illness than to cure it, but preventive treatment of all animals would be a waste of water and possibly cause unnecessary spreading of chemicals. It is usually possible to determine which animals have a higher risk to become ill, such as heifers. An animal which has had a foot rot infection usually develops immunity against foot rot, but heifers have no such immunity. It is therefore important to reduce the risk of infection for the heifers by regularly cleaning away any bacteria from their hooves. The same applies if an animal from a group of animals having no infections is moved to a group of animals where there are infections.

If there are animals with chronic digital dermatitis in a particular group, spread of the infection can be prevented by cleaning the hooves of the other animals in this group, so that any bacteria can be cleaned away from their hooves before it causes an infection. If it can be detected when a chronic digital dermatitis infection becomes acute, it is enough to clean the hooves of the other animals during this stage, since a chronic infection which is not acute will not spread any bacteria.

By selecting the subset of the plurality of animals housed in the animal arrangement based also on the risk for each of the plurality of animals to become ill, particularly by selecting as the subset those animals having the highest risks to become ill, and by spraying a cleaning liquid, e.g. water, or a sanitizing liquid, onto one or more of the hooves of these animals only, a selective and preventive treatment of some animals may be made to help reduce the incidence of digital dermatitis and foot rot in a herd.

In another embodiment, the hoof status includes whether the animal has a hoof illness, and the control arrangement is configured to select the subset of the plurality of animals housed in the animal arrangement based on the information for each of the plurality of animals regarding whether that animal has the hoof illness, particularly by selecting as the subset those animals having the hoof illness, wherein the hoof illness may e.g. be digital dermatitis or foot rot. The liquid may be a medicating liquid, which e.g. comprises chemicals and/or antibiotics.

Hereby, the medicating liquid is only used when necessary, i.e. when treating ill animals, while healthy animals are not given the medication. Too frequent use of a medication is not recommended since bacteria may become resistant to the medication, and also because it may spread into the environment, which is undesirable. Different types of chemicals and/or antibiotics may be sprayed onto the hooves. For treatment of digital dermatitis which has not yet developed into a chronic infection, antibiotics may be sprayed directly onto the infected area. Chronic digital dermatitis may be prevented from erupting into an acute infection if a disinfectant such as 4Hooves is regularly sprayed onto the infected area. For treatment of foot rot, penicillin, tetracycline or other antibacterial medicines may be sprayed directly onto the infected area. It is thus advantageous to be able to direct the spray from either the front or the back onto the hooves.

The animal specific information, on which the selection of the subset of the plurality of animals housed in the animal arrangement is based, may include also animal specific information manually entered into the database by a dairy farmer.

A second aspect, not according to the invention, refers to a method of hoof treatment in an animal arrangement, according to which each of a plurality of animals housed in the animal arrangement is identified, the hoof status for each of the identified animals is determined using an automatic detector arrangement, an information database of the animal arrangement, which database comprises animal specific information for a plurality of animals housed in the animal arrangement, is updated with respect to the hoof status for each of the idenified animals, a subset of the plurality of animals housed in the animal arrangement is automatically selected based on the animal specific information, and a liquid is automatically sprayed onto one or more of the hooves of only the animals of the selected subset of the plurality of animals by an automatic hoof spraying device, while they are stationary in a stall of the animal arrangement.

The second aspect may be modified to encompass method steps for performing any of the functions provided by devices, arrangements, or other equipment as disclosed in the various embodiments of the first aspect.

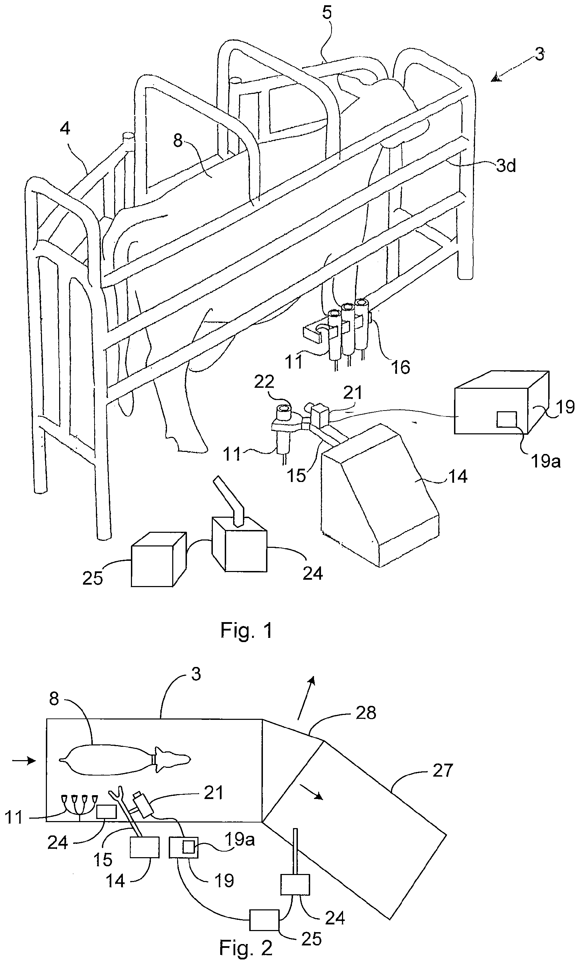

Further characteristics and advantages will be evident from the following detailed description of embodiments given hereinafter and the accompanying Figs. 1-3, which are given by way of illustration only, and are thus not limitative.

### BRIEF DESCRIPTION OF THE DRAWINGS

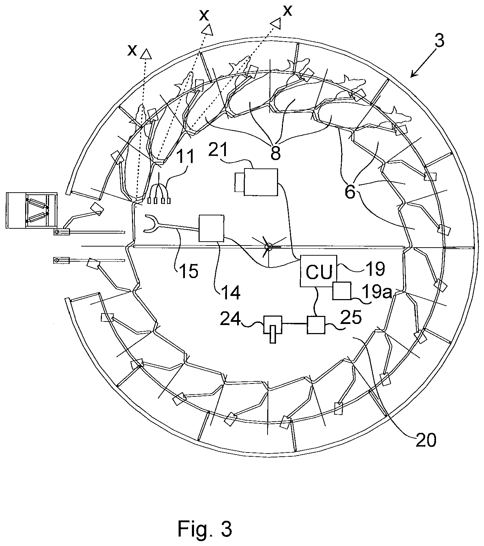

Figs. 1-3 display each a schematically outlined milking system including an automatic hoof treatment arrangement according to a respective embodiment. Fig. 1 is a perspective view while Figs. 2 and 3 are each a top view.

Identical reference numerals are used throughout the figures to denote identical or similar components, portions, details and the like of the various embodiments.

### DETAILED DESCRIPTION OF EMBODIMENTS

Fig. 1 shows an automated milking system or station 3 in an animal arrangement, in which an automatic hoof treatment arrangement according to an embodiment is implemented. The automated milking station 3 may be a voluntary milking station 3 comprising an enclosure having an inlet gate 4 and an outlet gate 5, which are both capable of being opened automatically.

The milking station 3 further comprises an automatic milking machine (not explicitly illustrated) including teat cups 11 connected to an end unit by means of milk lines (only the portions attached to the teat cups 11 are shown in Fig. 1). The milking station further includes a milking robot 14 having a movable robot arm 15 provided with a camera 21 and a gripper 22. The milking robot 14 is arranged to automatically attach the teat cups 11 of the milking machine to the teats of an animal 8 present in the milking station 3 prior to milking. In Fig. 1 three of the teat cups 11 are arranged in a teat cup rack or magazine 16, whereas the fourth one is held by the gripper 22 of the robot arm 15.

Further, the milking station 3 comprises an identification system (not illustrated) arranged to identify an animal approaching the milking station 3, and a milking station controller 19, which is responsible for controlling the milking station 3, which *inter alia* includes the initiation of various activities in connection with milking such as e.g. opening and closing of the gates 4 and 5, and control of the milking machine and the milking robot 14. The milking station controller 19 may further comprise an information database 19a, which comprises animal specific information for a plurality of animals housed in the animal arrangement.

The camera 21, which may be a three-dimensional camera, is operatively connected to the milking station controller 19 and is arranged to visualize the teats of the animals 8 present in the milking station 3, thereby enabling the milking station controller 19 to determine the positions of the teats of the animal 8 and to control the robot 14 to move the teat cups 11 to the teats of the animals 8 present in the milking station 3.

Furthermore, the hoof treatment arrangement comprises an automatic detector arrangement 21 configured to determine the hoof status for each of the animals, wherein the control arrangement 25 is operatively connected to the automatic detector arrangement to obtain information regarding the hoof status for each of the animals and to update the information database 19a with respect to the hoof status for each of the animals.

In one version, the detector arrangement is constituted by the camera 21 on the robot arm 15 and the milking station controller 19. The camera 21 is configured to record images of one or more hooves the animal 8, and the milking station controller 19 is configured to process the recorded images and to detect whether the animal 8 has the hoof illness. To this end, the milking station controller 19 may be provided with suitable image processing software. The robot 14 is typically configured to move the camera to appropriate positions to enable the camera 21 to record the images of one or more hooves the animal 8 while the animal 8 is stationary in the milking station 3, e.g. after the robot 14 has attached the teat cups 11 to the teats of the animal. The camera 21 may be configured to record the images of one or more of the hooves, e.g. the hooves of the hind legs, of each animal.

The hoof treatment arrangement for the milking station 3 comprises an automatic spray device 24 arranged to spray a liquid onto hooves of animals while they are present and stationary in the milking station 3, and a control arrangement 25 operatively connected to the automatic spray device 24 to control the automatic spray device 24. The automatic spray device 24 is only shown schematically, but may be either a spray device fixedly mounted in the stall, such as e.g. the one described in EP 1384400, or a spray device mounted on a robot arm, either the robot arm 15 or a separate robot arm, in order for the automatic spray device 24 to be moved to appropriate positions for the hoof spraying. The automatic spray device 24 may have one or more spraying orifices or nozzles.

Further, the control arrangement 25 of the hoof treatment arrangement is operatively connected to the information database 19a of the milking station controller 19 in order to be capable of retrieving the animal specific information for the animals housed in the animal arrangement. The control arrangement 25 may be implemented as a separate computer, microprocessor, circuit, or the like, or be integrated with the milking station controller 19 in a single computer.

The control arrangement 25 of the hoof treatment arrangement is configured (i) to select a subset of the animals housed in the animal arrangement based on the animal specific information retrieved from the database 19a and (ii) to control the automatic spray device 24 to spray a liquid onto one or more of the hooves of only the animals of the selected subset of the animals.

According to the invention, the animal specific information for the animals housed in the animal arrangement further comprises information regarding the risk for each of the animals to become ill, which is determined based on e.g. age, race, behavior, lactation status, illness history, or illness history of related animals, and the control arrangement 25 is configured to select the subset of the animals housed in the animal arrangement based also on the risk for each of the animals to become ill, particularly by selecting as the subset those animals having the highest risks to become ill. The risk for an animal to become ill may e.g. be calculated based on the stated parameters, and if the risk is above a certain threshold, the animal may be selected as part of the subset. The liquid is a cleaning liquid, such as e.g. water, or a sanitizing liquid.

In an alternative embodiment, the hoof status for the animals housed in the animal arrangement comprises information for each of the animals regarding whether that animal has a hoof illness, and the control arrangement 25 is configured to select the subset of the animals housed in the animal arrangement based on the information for each of the animals regarding whether that animal has the hoof illness, particularly by selecting as the subset those animals having the hoof illness. The liquid may here be a medicating liquid, such as e.g. a liquid comprising antibiotics. The hoof illness may e.g. be digital dermatitis or foot rot.

The automatic spray device 24 may be configured to spray a cleaning liquid, such as e.g. water, onto the hooves of each of the animals prior to the detector arrangement determining the hoof status for that animal.

Fig. 2 illustrates a further embodiment which differs from the embodiment of Fig. 1 in that the automatic hoof treatment arrangement is not implemented in, but instead in proximity of, an automated milking system. Here, a treatment stall 27, at which the automatic spray device 24 and the control arrangement 25 of the automatic hoof treatment arrangement are arranged, is located separate from the milking station 3. A gate arrangement 28, or similar, is arranged to automatically guide the animal 8 to the treatment stall 27 after having been milked in the milking station 3. Since the animal is sent directly from the milking station 3 to the treatment stall 27, there is no need for a separate identification system - instead the identification system in the milking station 3 (not illustrated) can be used.

As in the previously illustrated embodiment, an automatic detector arrangement 21 configured to determine the hoof status for the animals may be provided. However, in the disclosed treatment stall no robots are provided, and therefore, if the automatic detector arrangement is to be based on existing equipment, detection has to take place in the milking station 3. Alternatively, an automatic detector arrangement is arranged in the treatment stall 27.

Note that the gate arrangement 28 may be arranged to only automatically guide animals selected to obtain a hoof treatment to the treatment stall 27 after having been milked in the milldng station 3. Other animals may be automatically guided elsewhere by the gate arrangement 28. This is of course only possible if the automatic detector arrangement is arranged prior to the gate arrangement 28, e.g. in the milking station 3.

In a yet further embodiment (not illustrated), the automatic hoof treatment arrangement is implemented in a stall of a feeding station, which the animals of the animal arrangement can visit.

Fig. 3 shows a rotary milking system 3, in which an automatic hoof treatment arrangement according to a further embodiment is implemented.

The rotary milking system 3 comprises a rotatable carousel or rotating platform 20, which forms the support for a plurality of milking stalls 6, which animals 8 may enter in a sequential order. The rotary milking station 3 comprises an identification system (not illustrated), which may e.g. be located at the entrance to the rotating platform 20, in the milking stalls 6, or at a fixed point which the rotating platform 20 passes during rotation. Each of the milking stalls 6 comprises milking equipment including teat cups 11 that are attached to the teats of the animal present in the milking stall prior to milking. For the sake of simplicity, teat cups 11 are illustrated only for one of the milking stalls 6. The rotary milking system 3 may be of parallel, tandem, or, as illustrated, herringbone configuration, wherein the longitudinal directions x of the milking stalls 6 and of the animals 8 present therein extends partly radially, partly circumferentially.

A robot 14 provided with a robot arm 15 is arranged to automatically attach teat cups 11 to the teats of the animals 8 present in the milking stalls 6 under the control of a milking station controller 19, which is operatively connected to the milking robot 14. The milking robot 14 is preferably stationary with respect to the rotating platform 20 of the rotary milking system 3. Alternatively, the milking robot 14 is movable back and forth in e.g. a circumferential direction. A three-dimensional camera 21 is operatively connected to the milking station controller 19 and is arranged to visualize the teats of the animal 8 present in each milking stall 6, thereby enabling milking station controller 19 to determine the positions of the teats of the animal 8 and to control the robot 14 to move the teat cups 11 to the teats of the animal 8 present in each milking stall 6. The milking station controller 19 comprises a database 19a comprising animal specific information for the animals.

The automatic hoof treatment arrangement comprises an automatic spray device 24 arranged to spray a liquid onto hooves of animals while they are stationary in a respective one of the milking stalls 6, and a control arrangement 25 operatively connected to the automatic spray device 24 to control the automatic spray device 24. The automatic spray device may be fixedly mounted on a post which the platform rotates past, or a spray device mounted on a robot arm 15. The robot arm 15 may be a part of a teat cleaning robot, a teat cup attachment robot or a teat spray robot.

The control arrangement 25 of the hoof treatment arrangement is operatively connected to the information database 19a and is configured (i) to select a subset of the animals visiting the rotary milking system based on the animal specific information and (ii) to control the automatic spray device 25 to spray a liquid onto one or more of the hooves of only the animals of the selected subset of the animals.

In other respects, the hoof treatment arrangement of Fig. 3 may be similar to the hoof treatment arrangement of Fig. 1.

It shall be appreciated by a person skilled in the art that the above disclosed embodiments are exemplary embodiments and may be modified or altered without departing from the scope of the invention defined by the appended patent claims.

## Claims

1. A hoof treatment arrangement arranged to spray a liquid onto hooves of animals (8) while they are stationary in a stall (3, 6, 27) of an animal arrangement, the hoof treatment arrangement comprising an identification system configured to identify animals (8), an automatic detector arrangement (21) configured to determine the hoof status for animals (8), an automatic spray device (24), and a control arrangement (25), wherein the control arrangement (25) is:
- operatively connected to an information database (19a) of the animal arrangement which comprises animal specific information for a plurality of animals housed in the animal arrangement;
- operatively connected to the identification system, to obtain information regarding the identity of each of the plurality of animals;
- operatively connected to the automatic detector arrangement (21), to obtain information regarding the hoof status for each of the identified animals;
- operatively connected to the automatic spray device (24), to control the automatic spray device (24);
- arranged to update the information database (19a) with respect to the hoof status for each of the identified animals; and
- configured (i) to select a subset of the plurality of animals housed in the animal arrangement based on the animal specific information and (ii) to control the automatic spray device (24) to spray a liquid onto one or more of the hooves of only the animals of the selected subset of the plurality of animals while they are stationary in the stall (3, 6, 27) of the animal arrangement; and wherein
- the hoof status includes the degree of cleanliness of the hooves; and
- the liquid is a cleaning or sanitizing liquid;
the arrangement **characterised in that**:
- the animal specific information further includes information regarding the risk for each of the plurality of animals to become ill, which is determined based on e.g. age, race, behavior, lactation status, illness history, or illness history of related animals;
- the control arrangement (25) is configured to select the subset of the plurality of animals housed in the animal arrangement based on the degree of cleanliness of the hooves combined with the risk for each of the plurality of animals to become ill, particularly by selecting as the subset those animals (8) having the highest risks to become ill where the degree of cleanliness is below a threshold.

2. The arrangement of claim 1 wherein the automatic detector arrangement comprises a camera (21) configured to record images of one or more hooves of each of the identified animals, and image processing means configured to process the recorded images and to determine the hoof status for each of the identified animals.

3. The arrangement of claim 2 wherein the camera (21) is carried by a robot arm (15) configured to move the camera (21) to appropriate positions to enable the camera (21) to record images of one or more hooves of each of the identified animals.

4. The arrangement of any one of the preceding claims wherein the automatic spray device (24) is carried by a robot arm (15) configured to move the automatic spray device (24) to appropriate positions in order for the automatic spray device (24) to spray the liquid onto one or more of the hooves of only the animals of the selected subset of the plurality of animals.

5. The arrangement of claim 3 or 4 wherein the stall of the animal arrangement is a stall (3) of an automated milking system, and the robot arm (15) is a part of a teat cup attachment robot.

6. The arrangement of claim 3 or 4 wherein the stall of the animal arrangement is a stall (6) of a rotary milking system (3) comprising a rotating platform (20) with a plurality of milking stalls (6) arranged thereon, each of which being provided with teat cups (11) and provided for housing a animal (8) during milldng thereof, and the robot arm (15) is a part of a teat cleaning robot, a teat cup attachment robot or a teat spray robot of the rotary milking system (3).

7. The arrangement of any one of claims 1-4 wherein the stall of the animal arrangement is a stall (27) of an animal treatment station arranged in proximity to a milldng system (3) of the animal arrangement, and into which each of the plurality of animals is passing prior, or subsequent, to the milking thereof.

8. The arrangement of any one of claims 1-4 wherein the stall of the animal arrangement is a stall of an animal feeding station of the animal arrangement.

9. The arrangement of any one of the preceding claims wherein the automatic detector arrangement (21) is configured to determine the hoof status for each of the identified animals while the animal (8) is stationary in said stall (3, 6, 27) of the animal arrangement.

10. The arrangement of claim 9 wherein the automatic spray device (24) is configured to spray a cleaning liquid, such as e.g. water, onto one or more hooves of each of the identified animals prior to the detector arrangement (21) determining the hoof status for that animal (8).

11. The arrangement of any of the preceding claims, wherein the liquid is water.

## Patentansprüche

1. Klauenbehandlungsanordnung, die angeordnet ist, um eine Flüssigkeit auf Klauen von Tieren (8) zu sprühen, während diese in einer Stallbox (3, 6, 27) einer Tieranordnung stationär sind, wobei die Klauenbehandlungsanordnung ein Erkennungssystem, das konfiguriert ist, um Tiere zu erkennen (8), eine automatische Detektoranordnung (21), die konfiguriert ist, um den Klauenzustand für Tiere (8) zu bestimmen, eine automatische Sprühvorrichtung (24) und eine Steueranordnung (25) umfasst, wobei die Steueranordnung (25) Folgendes ist:
- wirkverbunden mit einer Informationsdatenbank (19a) der Tieranordnung, die tierspezifische Informationen für mehrere Tiere umfasst, die in der Tieranordnung untergebracht sind;
- wirkverbunden mit dem Erkennungssystem, um Informationen in Bezug auf die Identität jedes der mehreren Tiere zu erhalten;
- wirkverbunden mit der automatischen Detektoranordnung (21), um Informationen in Bezug auf den Klauenzustand für jedes der erkannten Tiere zu erhalten;
- wirkverbunden mit der automatischen Sprühvorrichtung (24), um die automatische Sprühvorrichtung (24) zu steuern;
- angeordnet, um die Informationsdatenbank (19a) in Bezug auf den Klauenzustand für jedes der erkannten Tiere zu aktualisieren; und
- konfiguriert (i), um eine Teilmenge der mehreren Tiere, die in der Tieranordnung untergebracht sind, basierend auf den tierspezifischen Informationen auszuwählen und (ii) um die automatische Sprühvorrichtung (24) zu steuern, um eine Flüssigkeit auf eine oder mehrere Klauen nur der Tiere der ausgewählten Teilmenge der mehreren Tiere zu sprühen, während sie stationär in der Stallbox (3, 6, 27) der Tieranordnung sind; und wobei
- der Klauenzustand den Reinheitsgrad der Klauen einschließt; und
- die Flüssigkeit eine Reinigungs- oder Desinfektionsflüssigkeit ist;
- die Anordnung **dadurch gekennzeichnet ist, dass**:
- die tierspezifischen Informationen ferner Informationen in Bezug auf das Risiko für jedes der mehreren Tiere einschließen, krank zu werden, was basierend auf z. B. Alter, Rasse, Verhalten, Laktationszustand, Krankheitsgeschichte oder Krankheitsgeschichte verwandter Tiere bestimmt wird;
- die Steuerungsanordnung (25) konfiguriert ist, um die Teilmenge der mehreren in der Tieranordnung untergebrachten Tiere basierend auf dem Reinheitsgrad der Klauen zusammen mit dem Risiko für jedes der Tiere, krank zu werden, auszuwählen, insbesondere durch Auswählen jener Tiere (8) als die Teilmenge, die das höchste Risiko aufweisen, krank zu werden, wenn der Reinheitsgrad unter einem Schwellenwert liegt.

2. Anordnung nach Anspruch 1, wobei die automatische Detektoranordnung eine Kamera (21), die konfiguriert ist, um Bilder von einer oder mehreren Klauen jedes der erkannten Tier aufzunehmen, und Bildverarbeitungsmittel umfasst, die konfiguriert sind, um die aufgenommenen Bilder zu verarbeiten und um den Klauenzustand für jedes der erkannten Tiere zu bestimmen.

3. Anordnung nach Anspruch 2, wobei die Kamera (21) von einem Roboterarm (15) getragen wird, der konfiguriert ist, um die Kamera (21) an geeignete Positionen zu bewegen, um es der Kamera (21) zu ermöglichen, Bilder von einer oder mehreren Klauen jedes der erkannten Tiere aufzunehmen.

4. Anordnung nach einem der vorhergehenden Ansprüche, wobei die automatische Sprühvorrichtung (24) von einem Roboterarm (15) getragen wird, der konfiguriert ist, um die automatische Sprühvorrichtung (24) an geeignete Positionen zu bewegen, damit die automatische Sprühvorrichtung (24) die Flüssigkeit auf eine oder mehrere der Klauen nur der Tiere der ausgewählten Teilmenge der mehreren Tiere zu sprühen.

5. Anordnung nach Anspruch 3 oder 4, wobei die Stallbox der Tieranordnung eine Stallbox (3) eines automatisierten Melksystems ist und der Roboterarm (15) ein Teil eines Zitzenbecherbefestigungsroboters ist.

6. Anordnung nach Anspruch 3 oder 4, wobei die Stallbox der Tieranordnung eine Stallbox (6) eines Rotationsmelksystems (3) ist, das eine rotierende Plattform (20) mit mehreren darauf angeordneten Melkständen (6) umfasst, wobei jeder dieser mit Zitzenbechern (11) versehen ist und zum Unterbringen eines Tieres (8) während des Melkens davon bereitgestellt ist, und wobei der Roboterarm (15) ein Teil eines Zitzenreinigungsroboters, eines Zitzenbecherbefestigungsroboters oder eines Zitzensprühroboters des Rotationsmelksystems (3) ist.

7. Anordnung nach einem der Ansprüche 1-4, wobei die Stallbox der Tieranordnung eine Stallbox (27) einer Tierbehandlungsstation ist, die in der Nähe eines Melksystems (3) der Tieranordnung angeordnet ist und in den jedes der mehreren Tiere vor oder im Anschluss an das Melken davon geführt wird.

8. Anordnung nach einem der Ansprüche 1-4, wobei die Stallbox der Tieranordnung eine Stallbox einer Tierfütterungsstation der Tieranordnung ist.

9. Anordnung nach einem der vorhergehenden Ansprüche, wobei die automatische Detektoranordnung (21) konfiguriert ist, um den Klauenzustand für jedes der erkannten Tiere zu bestimmen, während das Tier (8) stationär in der Stallbox (3, 6, 27) der Tieranordnung ist.

10. Anordnung nach Anspruch 9, wobei die automatische Sprühvorrichtung (24) konfiguriert ist, um eine Reinigungsflüssigkeit, wie etwa z. B. Wasser, auf eine oder mehrere Klauen jedes der erkannten Tiere zu sprühen, bevor die Detektoranordnung (21) den Klauenzustand für dieses Tier (8) bestimmt.

11. Anordnung nach einem der vorangehenden Ansprüche, wobei die Flüssigkeit Wasser ist.

## Revendications

1. Dispositif de traitement de sabot disposé pour pulvériser un liquide sur des sabots d'animaux (8) alors qu'ils sont stationnaires dans une stalle (3, 6, 27) d'une installation pour animaux, le dispositif de traitement de sabot comprenant un système d'identification configuré pour identifier des animaux (8), un dispositif de détecteur automatique (21) configuré pour déterminer l'état du sabot pour des animaux (8), un dispositif de pulvérisation automatique (24) et un dispositif de commande (25), le dispositif de commande (25) étant :
- connecté de manière opérationnelle à une base de données d'informations (19a) de l'installation pour animaux qui comprend des informations spécifiques à l'animal pour une pluralité d'animaux hébergés dans l'installation pour animaux ;
- connecté de manière opérationnelle au système d'identification, pour obtenir des informations concernant l'identité de chacun de la pluralité d'animaux ;
- connecté de manière opérationnelle au dispositif de détecteur automatique (21), pour obtenir des informations concernant l'état du sabot pour chacun des animaux identifiés ;
- connecté de manière opérationnelle au dispositif de pulvérisation automatique (24), pour commander le dispositif de pulvérisation automatique (24) ;
- disposé pour mettre à jour la base de données d'informations (19a) en ce qui concerne l'état du sabot pour chacun des animaux identifiés ; et
- configuré (i) pour sélectionner un sous-ensemble de la pluralité d'animaux hébergés dans l'installation pour animaux en fonction des informations spécifiques et (ii) afin de commander le dispositif de pulvérisation automatique (24) pour pulvériser un liquide sur un ou plusieurs des sabots de seulement les animaux appartenant au sous-ensemble sélectionné de la pluralité d'animaux alors qu'ils sont stationnaires dans la stalle (3, 6, 27) de l'installation pour animaux ; et
- l'état du sabot comportant le degré de propreté des sabots ; et
- le liquide étant un liquide de nettoyage ou de désinfection ;
le dispositif **caractérisé en ce que** :
- les informations spécifiques à l'animal comportent en outre des informations sur le risque de maladie pour chacun de la pluralité d'animaux, qui est déterminé en fonction de, par exemple, l'âge, la race, le comportement, l'état de la lactation, les antécédents pathologiques, ou les antécédents pathologiques d'animaux apparentés ;
- le dispositif de commande (25) est configuré pour sélectionner le sous-ensemble de la pluralité d'animaux hébergés dans l'installation pour animaux en fonction du degré de propreté des sabots combiné au risque de maladie pour chacun de la pluralité d'animaux, notamment en sélectionnant comme sous-ensemble les animaux (8) présentant le risque le plus élevé de maladie lorsque le degré de propreté est inférieur à un certain seuil.

2. Dispositif selon la revendication 1, dans lequel le dispositif de détecteur automatique comprend une caméra (21) configurée pour enregistrer des images d'un ou plusieurs sabots de chacun des animaux identifiés, et des moyens de traitement d'image configurés pour traiter les images enregistrées et pour déterminer l'état du sabot pour chacun des animaux identifiés.

3. Dispositif selon la revendication 2, dans lequel la caméra (21) est portée par un bras de robot (15) configuré pour déplacer la caméra (21) à des positions appropriées afin de permettre à la caméra (21) d'enregistrer des images d'un ou plusieurs sabots de chacun des animaux identifiés.

4. Dispositif selon l'une quelconque des revendications précédentes, dans lequel le dispositif de pulvérisation automatique (24) est porté par un bras de robot (15) configuré pour déplacer le dispositif de pulvérisation automatique (24) à des positions appropriées afin que le dispositif de pulvérisation automatique (24) pulvérise le liquide sur un ou plusieurs des sabots de seulement les animaux appartenant au sous-ensemble sélectionné de la pluralité d'animaux.

5. Dispositif selon la revendication 3 ou 4, dans lequel la stalle de l'installation pour animaux est une stalle (3) d'un système de traite automatisé, et le bras de robot (15) est une partie d'un robot de fixation du gobelet trayeur.

6. Dispositif selon la revendication 3 ou 4, dans lequel la stalle de l'installation pour animaux est une stalle (6) d'un système de traite rotatif (3) comprenant une plate-forme rotative (20) avec une pluralité de stalles de traite (6) disposée sur celui-ci, chacune d'elles étant pourvue de gobelets trayeurs (11) et pourvue pour héberger un animal (8) pendant sa traite, et le bras de robot (15) fait partie d'un robot de nettoyage de trayons, d'un robot de fixation de gobelet trayeur ou d'un robot de pulvérisation de trayons du système de traite rotatif (3).

7. Dispositif selon l'une quelconque des revendications 1 à 4, dans lequel la stalle de l'installation pour animaux est une stalle (27) d'un poste de traitement des animaux disposée à proximité d'un système de traite (3) de l'installation pour animaux, et dans laquelle chacun de la pluralité d'animaux passe avant ou après sa traite.

8. Dispositif selon l'une quelconque des revendications 1 à 4, dans lequel la stalle de l'installation pour animaux est une stalle d'un poste d'alimentation animale de l'installation pour animaux.

9. Dispositif selon l'une quelconque des revendications précédentes, dans lequel le dispositif de détecteur automatique (21) est configuré pour déterminer l'état du sabot pour chacun des animaux identifiés alors que l'animal (8) est stationnaire dans ladite stalle (3, 6, 27) de l'installation pour animaux.

10. Dispositif selon la revendication 9, dans lequel le dispositif de pulvérisation automatique (24) est configuré pour pulvériser un liquide de nettoyage, tel que par exemple l'eau, sur un ou plusieurs sabots de chacun des animaux identifiés avant que le dispositif de détecteur (21) détermine l'état du sabot pour cet animal (8).

11. Dispositif selon l'une quelconque des revendications précédentes, dans lequel le liquide est de l'eau.
